# EUROPEAN PATENT APPLICATION

(11) **EP 0 533 432 A1**
(43) Date of publication of application: **24.03.1993**
(21) Application number: 92308382.8
(22) Date of filing: 15.09.1992
(51) Int. Cl.: A61M 1/00, A61B 17/32

(54) **Catheter device**

(30) Priority: 17.09.1991 JP 236565/91
(71) Applicant: Yamauchi, Teiyu, Sumida-ku, Tokyo (JP); Furui, Shigeru, Tokorozawa-shi, Saitama (JP); KABUSHIKI KAISHA CLINICAL SUPPLY, Hashima-gun, Gifu (JP)
(72) Inventor: Yamauchi, Teiyu, Honjo-City House 701, Tokyo (JP); Furui, Shigeru, Tokorozawa-shi, Saitama (JP)
(74) Representative: BATCHELLOR, KIRK & CO.

(57) **Abstract**

A catheter device 11 for removal of physiological substances such as a thrombus, a hematoma, pus, or phlegm creates suction by directing a forced flow of a pressurized fluid toward a discharge port 10 of a catheter tube 11. A suction port 12 at or near a front end draws the physiological substances into the catheter tube. The forced flow of fluid tends to break up and dilute the physiological substances to ease their discharge through the discharge port. Embodiments are disclosed in which the fluid flow is created by a U-shaped fluid supply tube 21 having an injection port 22 directed toward the discharge port. The U-shaped tube may be fully inside the catheter tube, or only its end may be inserted into the catheter tube. In a further embodiment, the fluid supply tube discharges the fluid toward the suction port, and into a cup-shaped shield. Liquid or gas is injected into the catheter tube near the suction port and toward the discharge aperture to create a suction force at the suction port 12.

## Description

The present invention relates to a catheter device and, more particularly, to a device for removing a blocking physiological substance e.g., a thrombus from a blood vessel, a hematoma or pus from an affected part, or clogged phlegm from an organ of a body.

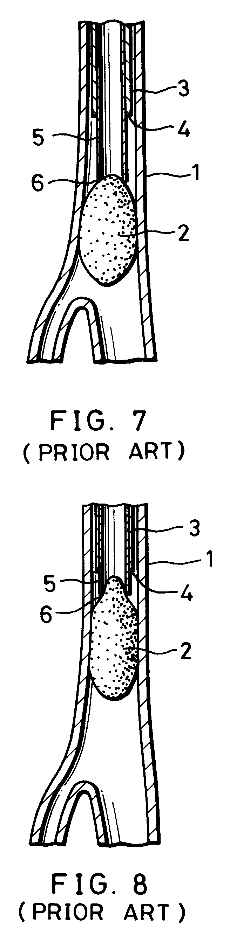

Conventionally, a thrombus is removed from a blood vessel by a device such as, for example, the one shown in Fig. 7. Such a device has a projecting end aperture 6 of a catheter tube 5 projecting from an end aperture 4 of a guide tube 3. Catheter tube 5 is inserted through guide tube 3 into a blood vessel 1 until projecting end aperture 6 is close to a thrombus 2 clogging blood vessel 1. Thrombus 2 is then removed by suction from the projecting end aperture 6 through catheter tube 5.

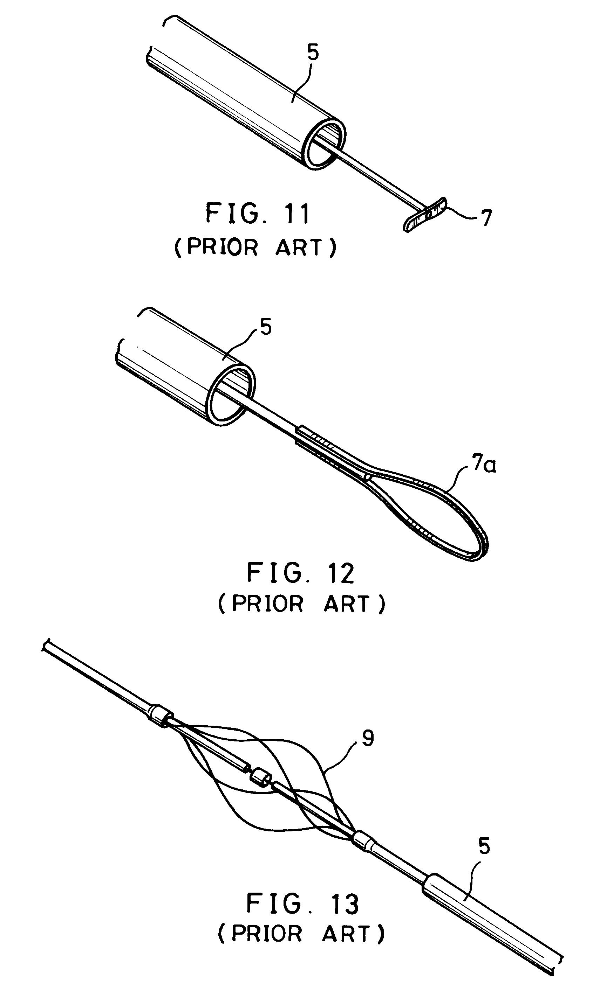

Other methods include those shown in Figs. 11, 12, and 13. Referring to Fig. 11, catheter tube 5 is provided with a rotating cutter 7 near its projecting end aperture 6. Rotating cutter 7 cuts thrombus 2, which is then removed by suction through catheter tube 5.

Referring to Fig. 12, catheter tube 5 is provided with a rotating cutter 7a near its projecting end aperture 6. Rotating cutter 7a cuts thrombus 2, which is similarly removed by suction through catheter tube 5.

Referring to Fig. 13, a rotating cutter 9 inside a blood vessel (not shown) cuts thrombus 2, which is also removed by suction through catheter tube 5.

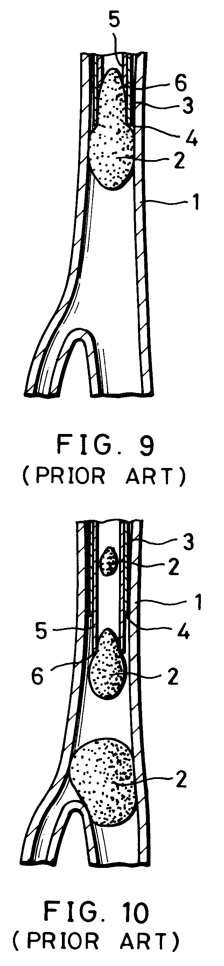

In the conventional method shown in Fig. 7, thrombus 2 is sucked into the end aperture 6 of catheter tube 5, as shown in Fig.8. Thrombus 2 is then cut and removed by suction as shown in Fig.10 by drawing catheter tube 5 into guide tube 3 as shown in Fig.9. This method presents problems: the removal of thrombus 2 is slow because of the number of separate steps that must be carried out, and it is difficult to determine whether all the pieces into which thrombus 2 has been cut have been sucked out of the blood vessel 1.

The methods shown in Figs. 11, 12 and 13, which require rotating cutter 7, 7a, or 9, require a rotating mechanism, which has a complicated and costly structure. The method shown in Fig. 13 presents further problems. Firstly, thrombus 2 is not discharged from the blood vessel but merely scattered within it. Secondly, the method shown in Fig.13 presents the potential danger of damaging the blood vessel.

An object of the present invention is to provide a catheter device for removing a thrombus, a hematoma, pus, or phlegm, by suction and which mitigates or even overcomes problems presented by prior devices.

A further object of the present invention is to provide a catheter device for removing a thrombus, a hematoma, pus, phlegm by suction using fluid pressure.

Another object of the present invention is to provide a catheter device for removing a thrombus, a hematoma, pus, or phlegm by suction using the ejecting action of a flow of fluid.

A further object of the present invention is to provide a catheter device for removing a thrombus, a hematoma, pus, or phlegm by suction that has a simple structure and which can remove viscous, potentially blocking physiological substances rapidly and reliably.

Briefly stated, the present invention provides a catheter device for removing physiological substances such as a thrombus, a hematoma, pus, or phlegm which creates suction by directing a forced flow of pressurized fluid toward a discharge port of a catheter tube, wherein a suction port at or near a front end draws the physiological substances into the catheter tube.

The forced flow of fluid tends to break up and dilute the physiological substances to ease their discharge through the discharge port. Embodiments are disclosed in which the fluid flow is created by a U-shaped fluid supply tube having an injection port directed toward the discharge port. The U-shaped tube may be fully inside the catheter tube, or only its end may be inserted into the catheter tube. In a further embodiment, the fluid supply tube discharges the fluid toward the suction port, and into a cup-shaped shield. The cup-shaped shield intercepts the fluid and redirects it toward the discharge port. The catheter tube can be inserted into an affected part or a clogged blood vessel with the suction port close to the physiological substance to be removed. Liquid or gas from the injection port of the fluid supply tube is injected i.e., forced under pressure into the catheter tube at or in the proximity of the suction port and toward the discharge aperture to create the suction at the suction port.

According to an embodiment of the invention, there is provided a catheter device comprising: a catheter tube having a front end and an aperture at a base end, a suction port in the catheter tube, the suction port being open near the front end, the aperture effective as a discharge port, a fluid supply tube, the fluid supply tube including at least a front end being inserted into the catheter tube, the fluid supply tube having a fluid injection port in proximity to the suction port, the fluid injection port being open toward the discharge port, and the fluid supply tube being effective, when fluid flows forcibly therethrough, for creating suction in the suction port and for ejecting the fluid, together with any viscous physiological substance sucked into and through the suction port, through the discharge port.

According to a preferred feature of the invention, there is provided a catheter device, which comprises: a catheter tube having a front end and a base end, the catheter tube having a suction port, the suction port being open in proximity to the front end, a discharge port at the base end, a fluid supply tube inserted into the catheter tube, the fluid supply tube having a fluid injection port in proximity to the suction port, a shield having a closed end in proximity to the suction port and an open end substantially enclosing the fluid injection port, and the shield being effective for deflecting a fluid discharged from the fluid injection port, whereby suction is created in the suction port when a fluid is forced into the fluid supply tube and injected through the fluid injection port, so as to be capable of removing a physiological substance by suction and discharging the physiological substance through the discharge port.

According to a further preferred feature of the invention, there is provided a method of removing a physiological substance from a body part, comprising the steps of: inserting a catheter tube into the body part with a suction port of the catheter tube placed in proximity to the physiological substance, forcing a fluid into the catheter tube via a fluid supply tube to create a suction in a suction port of the catheter tube, causing the physiological substance to enter the catheter tube by suction, and discharging the physiological substance through a discharge aperture of the catheter tube.

According to a still further preferred feature of the invention, there is provided a catheter comprising: a catheter tube, a suction port in the catheter tube, a discharge port in the catheter tube, means for injecting a pressurized fluid into the catheter tube, and the means for injecting including means for directing a flow of the pressurized fluid toward the discharge port, whereby a suction is created in the suction port.

In order that the invention may be illustrated, more easily understood and readily carried into effect, embodiments thereof will now be described purely by way of non-limiting example only, with reference to the accompanying drawings and wherein:
Fig. 1 is a cross section of a catheter device according to an embodiment of the present invention,
Fig. 2 is a cross section of a catheter device according to another embodiment of the present invention,
Fig. 3 is a cross section of a catheter device according to yet another embodiment of the present invention,
Fig. 4 is a cross section of a catheter device according to a further embodiment of the present invention,
Fig. 5 is a cross section of a catheter device according to a still further embodiment of the present invention,
Fig. 6 is a cross section of a catheter device according to an even further embodiment of the present invention,
Fig. 7 is a cross section of a conventional catheter device,
Fig. 8 and 9 are cross sections showing use of the conventional catheter device of Fig. 7,
Fig. 10 is a cross section of another conventional catheter device.
Fig. 11 is a perspective view of a further conventional catheter device,
Fig. 12 is a perspective view of an even further conventional catheter device, and
Fig. 13 is a perspective view of yet another conventional catheter device.

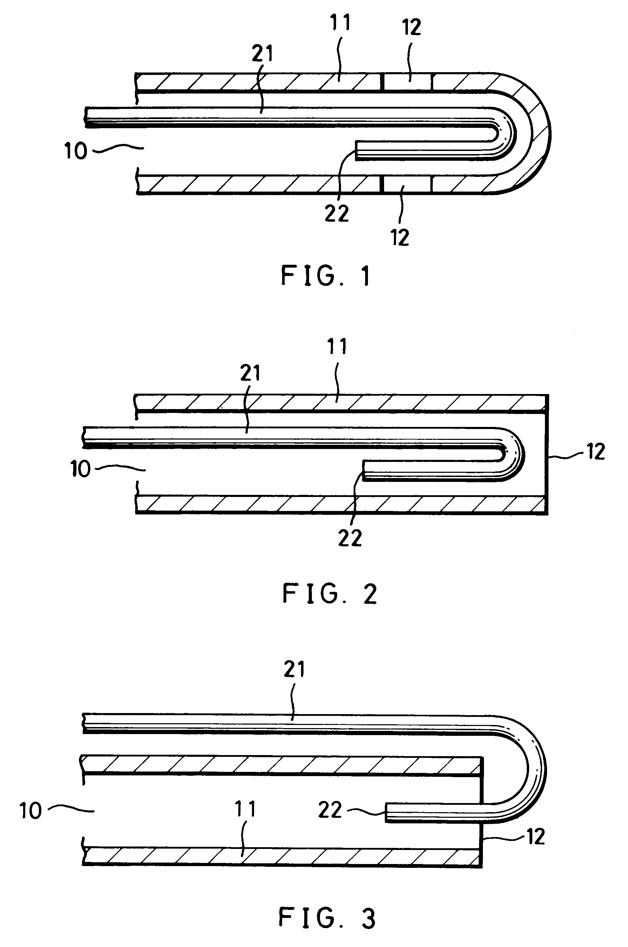

Referring to Fig. 1, a catheter tube 11 has a opening near a front end that serves as a suction port 12 and a base end aperture 10 that serves as a discharge port. Catheter tube 11 is formed from a polymer such as polyamide, TEFLON*, polyethylene, or urethane-polyamide, or a metal such as platinum-iridium, stainless steel, or alloys of these metals. The outer diameter of catheter tube 11 is approximately 1 - 4 mm.
*Registered Trademark

Where catheter tube 11 is formed from a polymer such as polyamide, polyethylene fluoride, polyethylene, polyurethane- polyamide, or polyethylene terephthalate, a contrast agent such as tungsten, bismuth trioxide, barium sulfate, etc., may be added if necessary in order to provide X ray absorption for monitoring the position of catheter tube 11 within a body.

A fluid supply tube 21 is inserted into catheter tube 11. In the vicinity of suction port 12 of catheter tube 11, fluid supply tube 21 is bent into a U shape. A fluid injection port 22 is located on the discharge side of suction port 12 opening toward the discharge port. Fluid supply tube 21 is formed from a polymer such as polyamide, polyethylene fluoride, polyurethane, or polyurethane-polyamide, or a metal such as platinum-iridium, stainless steel, or alloys of these metals.

Fluid supply tube 21 is configured to permit liquid or gas, such as, for example, isotonic sodium chloride solution or carbon dioxide, to be fed thereinto under pressure and injected through fluid injection port 22 so that an ejecting action of the injection flow from fluid injection port 22 creates a suction force as the suction port 12 of catheter tube 11. The liquid or gaseous fluid can be injected from fluid injection port 22 at the rate of approximately 1 - 50 cc per second, for example, where the inner diameter of catheter tube 11 is 1 mm - 3 mm.

Catheter tube 11 is inserted into a body so that its suction port 12 reaches the vicinity of the blocking substance e.g., thrombus, hematoma, pus, or phlegm to be removed. Then liquid or gas under pressure is fed into the fluid supply tube 21 and injected from fluid injection port 22 of fluid supply tube 21 toward the discharge end 10, the fluid injection port 22 being located near the discharge side of suction port 12. The thrombus, hematoma, pus, or phlegm is sucked through the suction port by ejecting action of the injected fluid. Although the physiological substance, such as the thrombus, hematoma, pus, or phlegm, may be viscous, the force of the injected fluid breaks it up and discharges it through the discharge port at base end aperture 10 of catheter tube 11.

It necessary, a pump may be used at base end aperture 10 of catheter 11 to create or improve the suction effect.

Referring to Fig. 2, in another embodiment of the present invention, the front end of catheter tube 11 is open, thereby serving as suction port 12 in this second embodiment. This arrangement contrasts with the first embodiment, where suction port 12 is at or near the front end of catheter tube 11. The configuration and operation of this second embodiment is otherwise the same as the first.

Referring to Fig. 3, in another embodiment of the present invention, fluid supply tube 21 is not completely inserted into catheter tube 11 but placed alongside catheter tube 11 so that the front end of fluid supply tube 21 is bent into a U-shape and inserted through suction port 12 into the open end of catheter tube 11. Fluid injection port 22 is close to suction port 12 and open toward the discharge end. The configuration and operation of this third embodiment is otherwise the same as the first.

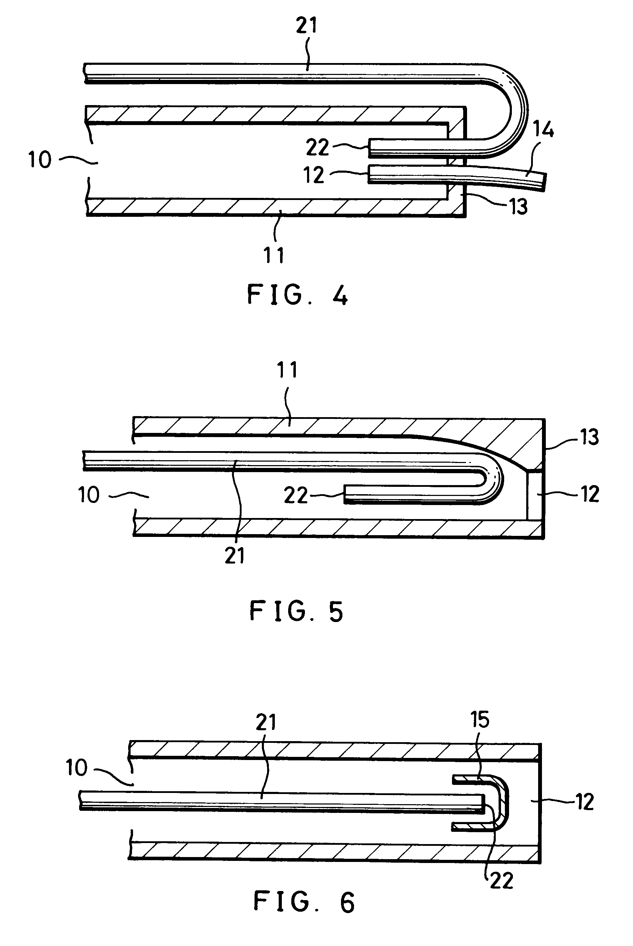

Referring to Fig. 4, in yet another embodiment of the present invention, fluid supply tube 21 is not fully inserted into catheter tube 11 but placed alongside catheter tube 11 so that the front end of fluid supply tube 21 is bent into a U-shape and inserted into catheter tube 11 through a closed front end 13 of catheter tube 11. A suction tube 14, a front end of which serves as suction port 12, is also inserted into closed front end 13 of catheter tube 11. The configuration and operation of this fourth embodiment is otherwise the same as the first.

Referring to Fig. 5, yet another embodiment of the present invention is configured similarly to the embodiment of Fig. 2, except the front end of catheter tube 11 is partially closed to form suction port 12, which becomes wider further inside catheter tube 11, thereby permitting a smoother suction effect.

Referring to Fig. 6, in still another embodiment a shield 15 in the shape of a cylinder closed at its bottom end is fixed inside catheter tube 11 in close proximity to suction port 12. Shield 15 is positioned so that its bottom end faces suction port 12. Fluid supply tube 21 is inserted in catheter tube 11 with its fluid injection port 22 open and facing the said bottom end of shield 15. Fluid is injected from fluid injection port 22 of fluid supply tube 21 toward shield 15. Injected fluid rebounds from shield 15 back toward the discharge end of catheter tube 11. Thus shield 15 serves as the injection port for the fluid, thereby producing the suction action.

A catheter device according to the present invention can remove by suction such viscous and potentially blocking physiological substances as thrombi, hematomas, pus, and phlegm by inserting a suction port of a catheter tube, into the vicinity of a thrombus or a hematoma (for example) at an affected part or a part clogged by pus or phlegm, and injecting, in the vicinity of the discharge end of the suction port, liquid or gas from an injection port of a fluid supply tube in the direction of the discharge side, thereby causing a suction action. Therefore the device is capable, through a simple structure, of removing viscous physiological fluid rapidly and reliably.

Having described preferred embodiments of the invention with reference to the accompanying drawings, it is to be understood that the invention is not limited to those precise embodiments, and that various changes and modifications may be effected therein by one skilled in the art without departing from the scope or spirit of the invention as defined in the appended claims.

## Claims

1. A catheter device comprising:
a catheter tube;
a suction port in said catheter tube;
a discharge port in said catheter tube;
means for injecting a pressurized fluid into said catheter tube; and
said means for injecting including means for directing a flow of said pressurized fluid toward said discharge port, whereby a suction is created in said suction port.

2. A catheter device as claimed in claim 1, wherein:
said means for injecting includes a fluid supply tube;
said fluid supply tube having at least an end portion in said catheter tube;
said end portion being directed toward said discharge port to create said suction.

3. A catheter device comprising:
a catheter tube having a front end and an aperture at a base end;
a suction port in said catheter tube;
said suction port being open near said front end;
said aperture effective as a discharge port;
a fluid supply tube;
said fluid supply tube including at least a front end inserted into said catheter tube;
said fluid supply tube having a fluid injection port in proximity to said suction port;
said fluid injection port being open toward said discharge port; and
said fluid supply tube being effective, when fluid flows forcibly therethrough, for creating suction in said suction port and for ejecting said fluid, together with physiological substances sucked through said suction port, through said discharge port.

4. A catheter device as claimed in any preceding claim, wherein:
said front end of said catheter tube is closed.

5. A catheter device as claimed in any one of claims 1 to 3, wherein:
said front end of said catheter tube is open or only partially closed to form said suction port.

6. A catheter device as claimed in any preceding claim, wherein:
only said front end of said fluid supply tube is inserted into said catheter tube.

7. A catheter device as claimed in any preceding claim other than claim 5, wherein:
said suction port of said catheter tube is a separate tube passing into said catheter.

8. A catheter device as claimed in any one of claims 1 to 6, wherein:
said suction port of said catheter tube is substantially tapered.

9. A catheter device as claimed in any preceding claim, wherein said means for directing a flow includes:
a fluid supply tube directing a flow of said pressurized fluid away from said discharge port;
a cup-shaped shield into which said flow is directed; and
said cup-shaped shield being effective for redirecting said flow toward said discharge port.

10. A catheter device, which comprises:
a catheter tube having a front end and a base end;
said catheter tube having a suction port;
said suction port being open in proximity to said front end;
a discharge port at said base end;
a fluid supply tube at least partially inserted into said catheter tube;
said fluid supply tube having a fluid injection port in proximity to said suction port;
a shield having a closed end in proximity to said suction port and an open end substantially enclosing said fluid injection port; and
said shield being effective for deflecting a fluid discharged from said fluid injection port, to enable a suction to be created in said suction port when a fluid is forced into said fluid supply tube and injected through said fluid injection port, to enable removal of a physiological substance by suction and discharge thereof through said discharge port.
